# EUROPÄISCHE PATENTSCHRIFT

(11) **EP 1 368 498 B1**
(45) Veröffentlichungstag und Bekanntmachung des Hinweises auf die Patenterteilung: **16.08.2006**
(21) Anmeldenummer: 02727215.2
(22) Anmeldetag: 12.03.2002
(51) Int. Cl.: C12Q 1/68, G01N 33/68, G01N 33/543

(54) **BIOSENSOR UND VERFAHREN ZUM ERFASSEN VON NUKLEINSÄUREN MITTELS MINDESTENS ZWEI EINHEITEN ZUM IMMOBILISIEREN VON NUKLEINSÄUREN**
BIOSENSOR AND METHOD FOR DETECTING NUCLEIC ACIDS BY MEANS OF AT LEAST TWO UNITS FOR IMMOBILIZING NUCLEIC ACIDS
BIOCAPTEUR ET PROCEDE POUR DETECTER DES ACIDES NUCLEIQUES AU MOYEN D'AU MOINS DEUX UNITES SERVANT A L'IMMOBILISATION DE CES ACIDES NUCLEIQUES

(30) Priorität: 16.03.2001 DE 10112778
(43) Veröffentlichungstag der Anmeldung: 10.12.2003
(73) Patentinhaber: SIEMENS AKTIENGESELLSCHAFT, 80333 München (DE)
(72) Erfinder: PAULUS, Christian, 82362 Weilheim (DE); THEWES, Roland, 82194 Gröbenzell (DE); SCHIENLE, Meinrad, 85579 Neubiberg (DE)
(74) Vertreter: Viering, Jentschura & Partner
(86) Internationale Anmeldenummer: PCT/DE2002/000867
(87) Internationale Veröffentlichungsnummer: WO 2002/074984

(56) Entgegenhaltungen:
- WO-A-99/51778
- DE-A- 19 926 457
- US-A- 5 972 692

## Beschreibung

Die Erfindung betrifft einen Biosensor und ein Verfahren zum Erfassen von Nukleinsäuren mittels mindestens zwei Einheiten zum Immobilisieren von Nukleinsäuren.

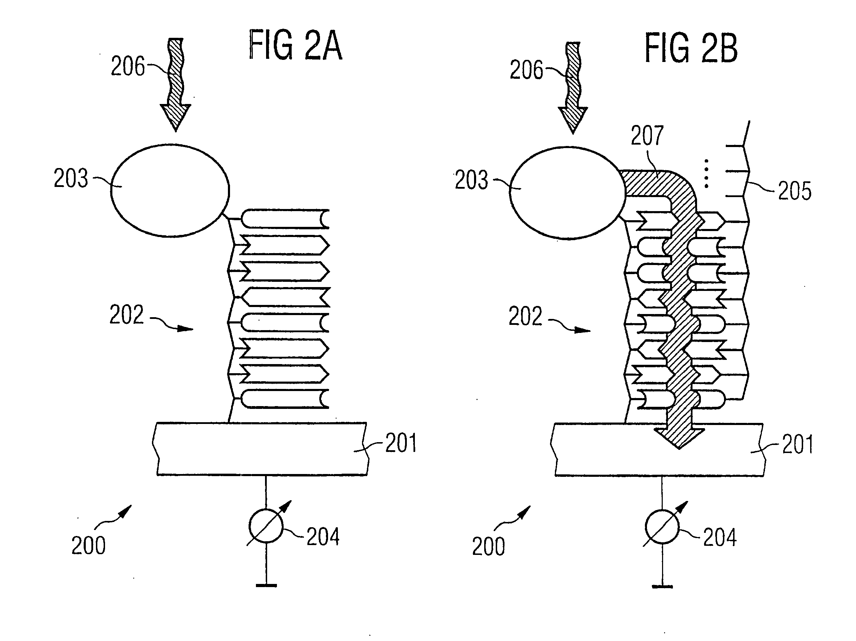

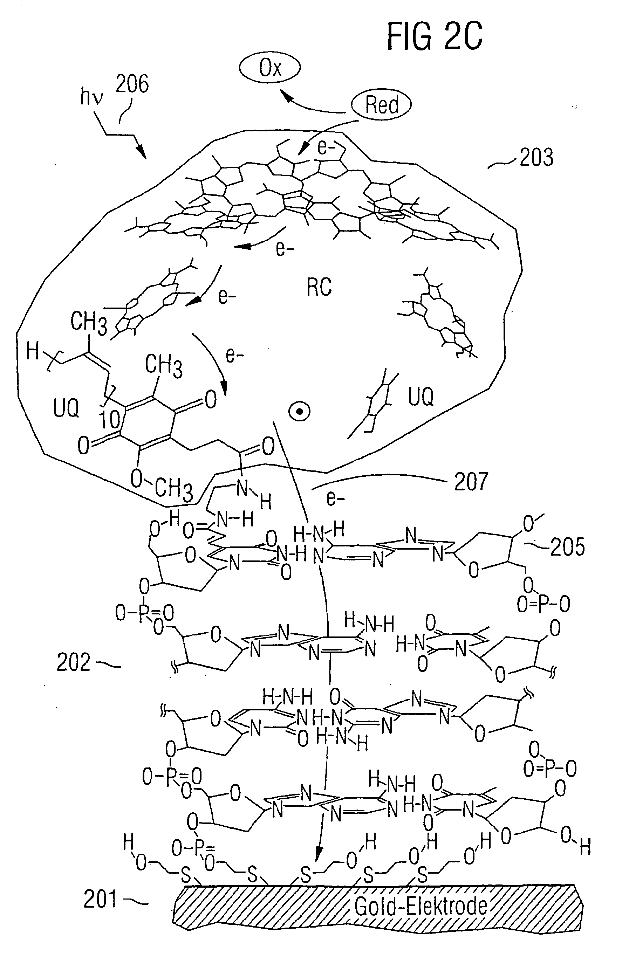

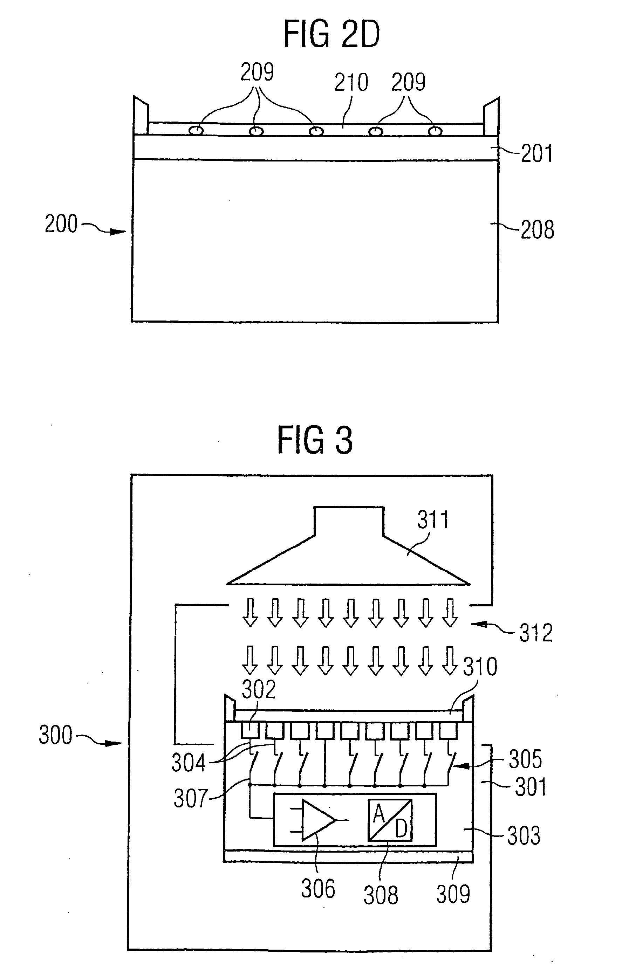

Aus [1] bis [5] ist ein elektrochemisches Verfahren zur Detektion von Nukleinsäure-Oligomer-Hybridisierungsereignissen, d.h. einfacher ausgedrückt, ein Verfahren zum Nachweis von Nukleinsäuren bekannt. Dieses Verfahren beruht, wie anhand Fig.2 näher erläutert, auf dem Unterschied der Leitfähigkeit von einzelsträngigen und doppelsträngigen Nukleinsäuren.

Ferner ist aus [6] eine Vorrichtung bekannt, bei der auf Teststellen Sondenmoleküle aufgebracht sind, die an eine zuvor bestimmte molekulare Targetstruktur binden können. Bei der Vorrichtung gemäß [6] werden nach Anbringen eines Signals bestimmte elektrische, mechanische oder optische Eigenschaften der Teststellen zum Nachweis der Bindung der Sondenmoleküle mit einer Targetstruktur herangezogen.

Darüber hinaus ist aus [7] eine Vorrichtung bekannt, bei der individuell adressierbare Mikrostellen zur Durchführung molekularbiologischer Reaktionen wie Nukleinsäure-Hybridisierungen verwendet werden können. Die Adressierbarkeit einer Mikrostelle wird durch eine unter ihr angeordnete Gleichstrom-Mikroelektrode erzielt. Deren Aufgabe ist es, elektrophoretische Anziehung oder Abstoßung von Bindungseinheiten oder Reaktanten bereitzustellen und somit die molekularbiologische Reaktion zu steuern.

**Fig.2a** und **2b** zeigen schematisch das Funktionsprinzip des Verfahrens gemäß [1] bis [5]. Ein Sensor 200 weist eine einheitlich ausgestaltete leitfähige Oberflächenschicht 201 auf, auf der auf bestimmten Positionen einzelsträngige Nukleinsäure/DNA-Moleküle 202 immobilisiert sind. Die einzelsträngigen Nukleinsäuremoleküle 202 dienen als Fängermoleküle für eine nachzuweisende Nukleinsäure und weisen eine redoxaktive Markierung 203 auf. Die Oberflächenschicht 201 ist ferner mit einem elektrischen Anschluss 204 versehen.

Der Sensor 200 wird in Kontakt mit einer zu untersuchenden Probe (nicht dargestellt), beispielsweise einem flüssigen Elektrolyten, gebracht.

Sind in dem Elektrolyten DNA-Stränge 205 mit einer Sequenz enthalten, die zu der Sequenz der DNA-Fängermoleküle 202 komplementär ist, so hybridisieren diese DNA-Stränge 205 mit den DNA-Fängermolekülen 202 (vgl. **Fig.2b**).

Eine Hybridisierung eines DNA-Fängermoleküls 202 und eines DNA-Strangs 205 findet nur dann statt, wenn die Sequenzen des jeweiligen DNA-Sondenmoleküls 202 und des entsprechenden DNA-Strangs 205 zueinander komplementär sind. Ist dies nicht der Fall, so findet keine Hybridisierung statt. Somit ist ein DNA-Sondenmolekül einer vorgegebenen Sequenz jeweils nur in der Lage einen bestimmten, nämlich den DNA-Strang mit jeweils komplementärer Sequenz zu binden, d.h. mit ihm zu hybridisieren.

Durch die Hybridisierung bilden sich, wie aus **Fig.2b** ersichtlich, doppelsträngige Nukleinsäuremoleküle.

Bei dem Verfahren nach [1] bis [5] wird nach einem nicht dargestellten Spülschritt Licht geeigneter Wellenlänge, wie durch den Pfeil 206 symbolisiert, auf den Sensor 200 gestrahlt. Die redoxaktive Markierung 203 setzt, durch das eingestrahlte Licht angeregt, kontinuierlich Elektronen frei. Falls die Markierung in einem doppelsträngigen Hybrid aus DNA-Fängermolekül 202 und zu erfassender Nukleinsäure 205 vorliegt, wirkt dieses doppelsträngige Hybrid als eine Art Elektronenpumpe und leitet Elektronen, wie durch den Pfeil 207 veranschaulicht, von der Markierung 203 zur leitfähigen Oberfläche 201, so dass an dieser ein Strom gemessen werden kann (vgl. **Fig.2b,c).** Falls jedoch keine Hybridisierung erfolgt, stellen einzelsträngige Fängermoleküle 202 näherungsweise einen Isolator dar, es fließt folglich kein Strom an der Oberfläche 201.

**Fig.2c,** die Fig.4 aus [3] entspricht, zeigt eine im molekularen Detail genauere Darstellung, bei der als redoxaktive Markierung ein photosynthetisches bakterielles Reaktionszentrum verwendet wird.

Der durch das doppelsträngige Moleküle bewirkte Stromfluss wird in [1] bis [5] mit einem Sensor 200 erfasst, der so gestaltet ist, dass auf einem isolierenden Trägermaterial 208 wie einem Glasplättchen/Muskovitplättchen ein durchgängiger, elektrisch leitender Goldfilm 201 aufgebracht wird (vgl. z.B. [3], Seite 20, Abschnitt "Modifizierte Oberflächen/Elektroden und Seite 54, 4. Absatz, Seite 56, Beispiel 2 sowie Seite 29, 1. Absatz). Dabei befinden sich eine oder mehrere sogenannter "Test-Sites", d.h. zuvor bestimmte Positionen oder Sensorfelder, an denen Fängermoleküle immobilisiert sind, auf dem Goldfilm. Ein solcher Sensor 200, der eine ganzflächig ausgebildete, mit verschiedenen Test-Sites 209 versehene Goldoberfläche 201 und ein Trägermaterial 208 aufweist, ist in **Fig.2d** gezeigt. **Fig.2d** zeigt ferner einen zu untersuchenden Analyten 210 auf dem Biosensor 200.

Ein Sensor gemäß [1] bis [5] arbeitet somit als rein passive Anordnung, d.h. als Sensor, der zur Erfassung/Auswertung des Signals an externe Messgeräte angeschlossen werden muss.

Als Vorteil ein solchen Sensors wird offenbar angesehen, dass die einzelnen Test-Sites nicht auf einzelnen, elektrisch voneinander isolierten und zum Anlegen eines Potentials und Auslesen einzeln ansteuerbare (Mikro)-Elektroden aufgebracht werden müssen (vgl. z.B. [3], Seite 55, erster Absatz). Eine Adressierung einzelner Sites erfolgt vielmehr durch gezielte Bestrahlung mit Licht geeigneter Wellenlänge.

Ein derartiger "passiver" Sensor mit externen Auswerteeinheiten erscheint jedoch aus mehreren Gründen als nachteilig.

Durch die ganzflächige Oberfläche entsteht eine hohe Kapazität, da stets alle Sensorfelder mit der externen elektrischen Ausleseeinheit verknüpft sind. Die bei dem Verfahren üblicherweise geringen Signale können durch diese vergleichsweise hohe Lastkapazität derart belastet sein, dass ihre Detektion mittels externer Strommessgeräte problematisch erscheint oder zumindest mit sehr großen Zeitkonstanten erfolgt.

Außerdem können Dunkelströme, d.h. parasitäre Ströme, von nicht durch Lichteinstrahlung ausgewählten Positionen auftreten. Diese Dunkelströme können dabei den Signalstrom übersteigen, was die maximal mögliche Anzahl von Sensorfeldern beschränken kann.

Des weiteren erscheint bei solchen Arrays das Übersprechen zwischen einzelnen Sensorfeldern ein Problem zu sein, insbesondere bei sehr kleinen nachzuweisenden Strömen.

Im Falle eines passiven, optisch adressierbaren Chips muss ein externes Lesegerät sehr kleine Analogsignale verarbeiten können. Dies ist in einer Umgebung mit weiteren elektrischen Geräte störanfällig.

Schließlich erscheint auch die Genauigkeit, mit der das Licht auf den Sensor eingestrahlt wird, problematisch. Das eingestrahlte Licht muss auf die ausgewählten, einzelnen Sensorfelder selektiv auffallen, ohne dabei die Nachbarfelder zu treffen. Dies verlangt eine aufwändige optische Anordnung beim Auslesen des Sensors.

Der vorliegenden Erfindung liegt daher das Problem zugrunde, ein alternatives Verfahren sowie einen alternativen Biosensor und eine Vorrichtung für die Erfassung von Nukleinsäuren bereitzustellen, die diese Nachteile nicht aufweisen.

Das Problem wird durch das Verfahren, den Biosensor sowie die Vorrichtung mit den Merkmalen gemäß den unabhängigen Patentansprüchen gelöst.

Das Verfahren für die Erfassung von Nukleinsäuren verwendet mindestens zwei Einheiten zum Immobilisieren von Nukleinsäuren. Dabei sind die mindestens zwei Einheiten zum Immobilisieren von Nukleinsäuren jeweils elektrisch leitend ausgestaltet und voneinander elektrisch isoliert.

Bei dem Verfahren werden die mindestens zwei Einheiten zum Immobilisieren von Nukleinsäuren mit als Fängermoleküle dienenden ersten Nukleinsäuremolekülen versehen. Die ersten Nukleinsäuremoleküle liegen als einzelsträngige Moleküle vor und können zweite zu erfassende Nukleinsäuremoleküle binden. Des weiteren werden die ersten als Fängermoleküle dienenden einzelsträngigen Nukleinsäuremoleküle mit einer redoxaktiven Markierung versehen, die ein detektierbares Signal erzeugen kann.

Die Markierung kann entweder vor der Immobilisierung der Fängermoleküle auf den mindestens zwei Einheiten zum Immobilisieren oder auch nach der Immobilisierung der Fängermoleküle auf den mindestens zwei Einheiten zum Immobilisieren geschehen.

Bei dem Verfahren wird eine zu untersuchende Probe mit den mindestens zwei Einheiten zum Immobilisieren von Nukleinsäuren in Kontakt gebracht, wobei die zu untersuchende Probe die zweiten zu erfassenden Nukleinsäuremoleküle enthalten kann. In der zu untersuchenden Probe enthaltene zweite Nukleinsäuremoleküle werden dabei an den Fängermolekülen gebunden, wodurch doppelsträngige Hybridmoleküle gebildet werden. Dann wird das Hybridisierungsereignis mittels eines durch die redoxaktive Markierung verursachten Signals unter Verwendung einer in einem Halbleiter-Chip integrierten elektrischen Erfassungsschaltung erfasst, die derart ausgestaltet ist, dass sie die Elektronen, die im Fall eines Sensorereignisses von der Markierung freigesetzt werden, und welche von dem doppelsträngigen Hybrid aus Fängermolekülen und zu erfassenden Nukleinsäuremolekülen von der Markierung zur Oberfläche einer jeweiligen Einheit zum Immobilisieren geleitet werden, erfasst.

Ein hier offenbarter Biosensor zum Erfassen von Nukleinsäuren weist mindestens zwei Einheiten zum Immobilisieren von Nukleinsäuren und eine elektrische Erfassungsschaltung auf. Die mindestens zwei Einheiten zum Immobilisieren von Nukleinsäuren des Biosensors sind jeweils elektrisch leitend ausgestaltet und voneinander elektrisch isoliert. Bei dem Biosensor sind die mindestens zwei Einheiten zum Immobilisieren von Nukleinsäuren mit als Fängermoleküle dienenden ersten Nukleinsäuremolekülen versehen, wobei die ersten Nukleinsäuremoleküle als einzelsträngige Moleküle vorliegen und zweite zu erfassende Nukleinsäuremoleküle binden können. Die ersten als Fängermoleküle dienenden einzelsträngigen Nukleinsäuremoleküle sind mit einer redoxaktiven Markierung versehen, die ein detektierbares Signal erzeugen kann. Die elektrische Erfassungsschaltung ist derart ausgestaltet, dass sie das Hybridisierungsereignis der Nukleinsäuremoleküle mit den Fängermolekülen mittels der Markierung erfasst, wobei die Erfassungsschaltung derart ausgestaltet ist, dass sie die Elektronen, die im Fall eines Sensorereignisses von der Markierung freigesetzt werden, und welche von dem doppelsträngigen Hybrid aus Fängermolekülen und zu erfassenden Nukleinsäuremolekülen von der Markierung zur leitfähigen Oberfläche einer jeweiligen Einheit zum Immobilisieren geleitet werden, erfasst.

Anschaulich ausgedrückt beruht die Erfindung darauf, dass im Gegensatz zu dem aus [1] bis [5] bekannten Erfassungsverfahren die Immobilisierung der Fängermoleküle und die Ausbildung der doppelsträngigen Hybridmoleküle nicht auf einer durchgängigen den elektrischen Strom leitenden Oberfläche (Elektrode) geschieht, sondern dass die Oberfläche unterteilt wird in Bereiche, die jeweils für sich leitfähig sind, jedoch nicht im elektrischen Kontakt zueinander stehen.

Bei dem Biosensor der Erfindung hat die Aufteilung in diese Bereiche mehrere Vorteile. Erstens wird dadurch jeder Bereich bzw. jede Position, die mit als Elektronenpumpen arbeitenden Molekülen versehen ist, nur mit der Kapazität eines einzelnen Sensorfeldes belastet. Zweitens kann dadurch die Messanordnung rauschärmer und störungssicherer ausgelegt werden.

Bei dem Verfahren der Erfindung ergibt sich durch diese Aufteilung der Vorteil, dass eine Einheit zum Immobilisieren oder mehrere dieser Einheiten für die individuelle Erfassung des Signals ausgewählt wird.

Diese eben diskutierten Bereiche der Sensorfläche werden hier auch als Einheiten zum Immobilisieren bezeichnet.

Unter "Einheit zur Immobilisierung" wird im Sinne der Erfindung eine Anordnung verstanden, die eine elektrisch leitende Oberfläche aufweist, auf der die Fängermoleküle immobilisiert werden können, d.h. an die die Fängermoleküle durch physikalische oder chemische Wechselwirkungen binden können. Diese Wechselwirkungen schließen hydrophobe, hydrophile, van der Waalsche oder ionische (elektrostatische) Wechselwirkungen und kovalente Bindungen ein. Beispiele für geeignete Oberflächen-Materialien, die für die mindestens eine Einheit zur Immobilisierung verwendet werden können, sind Metalle wie Gold oder Palladium oder elektrisch leitfähige Polymere. Eine Einheit zum Immobilisieren stellt in der vorliegenden Erfindung eine Elektrode oder einen Bestandteil einer Elektrode dar.

Die Immobilisierung auf einer Einheit kann in der Weise erfolgen, dass die gesamte Oberfläche einer Einheit zum Immobilisieren mit Fängermolekülen versehen wird. Es ist jedoch auch möglich, die Immobilisierung selektiv auf einzelne Bereiche/Punkte ("spots") einer Einheit zum Immobilisieren zu beschränken. Um letzteres zu erreichen, kann die Einheit zum Immobilisieren entsprechend ausgestaltet sein, z.B. durch chemisch für die Immobilisierung aktivierte Bereiche.

Der Begriff "redoxaktive Markierung" wird hier in der Bedeutung gebraucht, die in [1] bis [5] dem Begriff "redoxaktive Einheit" zukommt und z.B. in [3] auf Seite 9, 3. Absatz; Seite 22, 4. Absatz bis Seite 23, 3. Absatz angegeben ist.

Dies bedeutet, dass im Sinne der vorliegenden Erfindung eine redoxaktive Markierung eine chemische Verbindung, Gruppe oder Einheit aus mehreren Molekülen ist, die die Eigenschaft besitzt, unter bestimmten äußeren Umständen an ein geeignetes Oxidationsmittel Elektronen abzugeben oder von einem geeigneten Reduktionsmittel aufzunehmen bzw. die Eigenschaft besitzt, unter bestimmten äußeren Umständen an einen geeigneten Elektronen-Akzeptor Elektronen abzugeben oder von einem geeigneten Elektronen-Donor Elektronen aufzunehmen.

Eine redoxaktive Markierung im Sinne der Erfindung umfasst folglich die in [1] bis [5] definierten chemisch induzierbaren oder photoinduzierbaren redoxaktiven Einheiten (vgl. die Definition der photoinduzierbar redoxaktiven Einheit in [3] auf Seite 10, 2. Absatz bis Seite 12, 2. Absatz bzw. der chemisch induzierbar redoxaktiven Einheit auf Seite 12, 2. Absatz, Seite 13, 1. Absatz), die (kovalent) über mindestens eine Bindung an ein als Fängermolekül dienendes Nukleinsäure-Einzelstrang-Molekül gebunden sind.

Beispiele für im vorliegenden Verfahren einsetzbare photoinduzierbare redoxaktive Markierungen sind folglich das photosynthetische bakterielle Reaktionszentrum (RC), Cyclophane oder ein wenigstens bimolekularer Elektronen-Donor/Elektronen-Akzeptor-Komplex (letzterer selbstverständlich in seiner Bedeutung gemäß [1] bis [5], siehe [3], Seite 14, 2. Absatz, Seite 15, 1. Absatz). Bei letztgenanntem Komplex kann der Elektronen-Donor und der Elektronen-Akzeptor des biomolekularen Elektronen-Donor/Elektronen-Akzeptor-Komplexes ein Charge-Transfer-Komplex oder ein Übergangsmetall-Komplex sein.

Beispiele für chemisch induzierbare redoxaktive Markierungen sind daher der Cytochrom-bc-Komplex, der Cytochrom-C₂-Komplex der Photosynthese betreibenden Bakterien oder chemisch induzierbare wenigstens biomolekularer Elektronen-Donor/Elektronen-Akzeptor-Komplexe wie geeignete Cyclophane (siehe [3], Seite 31, 2. Absatz).

Allgemein wird bei dem hier beschriebenen Verfahren das durch die redoxaktive Markierung erzeugte Signal zur Erfassung von Nukleinsäuren herangezogen. Vorzugsweise geschieht die Erfassung durch Messung eines Stromflusses, eines Widerstands oder einer Leitfähigkeit.

In einer bevorzugten Ausführung des Verfahrens wird eine Einheit zum Immobilisieren zum individuellen Erfassen des Signals ausgewählt.

Unter Nukleinsäuren werden hier DNA-Moleküle, RNA-Moleküle, PNA-Moleküle auch kürzere Fragmente wie Oligonukleotide mit z.B. 10 bis 40 Basenpaaren (bp) verstanden. Die Nukleinsäuren können doppelsträngig sein, jedoch auch zumindest einzelsträngige Bereiche aufweisen oder, zum Beispiel durch vorangehende thermische Denaturierung (Strangtrennung) für ihren Nachweis, als Einzelstränge vorliegen. Die Sequenz der zu erfassenden Nukleinsäuren kann dabei zumindest teilweise oder vollständig vorgegeben, d.h. bekannt sein.

Wenn DNA-Moleküle (Nukleinsäuren oder Oligonukleotide) einer vorgegeben Nukleotidsequenz mit dem hier beschriebenen Verfahren erfasst werden, so werden sie vorzugsweise in einzelsträngiger Form erfasst, d.h. sie werden ggf. vor der Erfassung durch Denaturierung wie vorstehend erläutert in Einzelstränge überführt. In diesem Fall werden als Fängermoleküle dann vorzugsweise DNA-Sondenmoleküle mit einer zu dem einzelsträngigen Bereich komplementären Sequenz verwendet. Die DNA-Sondenmoleküle können wiederum Oligonukleotide oder auch längere Nukleotidsequenzen aufweisen, solange diese keine der intermolekularen Strukturen ausbilden, die eine Hybridisierung des Sondenmoleküls mit der zu erfassenden Nukleinsäure verhindern.

In einer Ausgestaltung des Verfahrens - und somit in einer Weiterentwicklung des aus [1] bis [5] bekannten Verfahrens werden nicht gebundene DNA-Sondenmoleküle, nachdem diese in Kontakt mit einer zu untersuchenden Probe gebracht wurden, von den mindestens zwei Einheiten zum Immobilisieren entfernt. Dadurch kann ein etwaiger Hintergrundstrom, der durch einzelsträngige markierte Sondenmoleküle hervorgerufen wird, zumindest verringert werden. Die Entfernung geschieht vorzugsweise, indem ein Enzym mit Nukleaseaktivität mit der Einheit zum Immobilisieren in Kontakt gebracht wird.

Für das Entfernen kann als Enzym mit Nukleaseaktivität mindestens einer der folgenden Stoffe verwendet wird:
- Nuklease aus Mung-Bohnen,
- Nuklease P1
- Nuklease S1, oder
- DNA-Polymerasen, die aufgrund ihrer
   5'→ 3' Exonukleaseaktivität oder ihrer
   3'→ 5' Exonukleaseaktivität imstande sind, einzelsträngige DNA abzubauen.

In einer bevorzugten Ausführungsform des Verfahrens weisen die Einheiten zum Immobilisieren Gold auf bzw. bestehen aus Gold.

Bei dem Verfahren sind in einer weiteren Ausgestaltung die mindestens zwei Einheiten zum Immobilisieren auf einem Halbleiter-Chip angeordnet. Vorzugsweise ist ein derartiger Halbleiter-Chip ein CMOS-Chip.

Anzumerken ist, dass es selbstverständlich möglich ist, mit dem vorliegenden Verfahren nicht nur eine einzige Art von Nukleinsäure in einer einzelnen Messreihe zu erfassen. Vielmehr können mehrere Nukleinsäuren gleichzeitig oder auch nacheinander erfasst werden. Dazu können auf den Einheiten zum Immobilisieren mehrere Arten von Fängermolekülen, von denen jedes eine (spezifische) Bindungsaffinität für eine bestimmte zu erfassende Nukleinsäure aufweist, gebunden werden, und/oder es können mehrere Einheiten zum Immobilisieren eingesetzt werden, wobei an jeder von diesen Einheiten nur eine Art von Fängermolekül gebunden wird. Bei diesen Mehrfachbestimmungen wird für jede zu erfassende Nukleinsäure vorzugsweise eine von den anderen Markierungen unterscheidbare Markierung verwendet, um so z.B. ungewollte Nebenreaktionen zu vermeiden.

Damit sie bei einer solchen Mehrfachbestimmung eingesetzt werden kann, weist der hier beschriebene Biosensor vorzugsweise mehrere, d.h. mehr als zwei, Einheiten zum Immobilisieren von Nukleinsäuren in einer regelmäßigen Anordnung auf.

In einer vorteilhaften Ausgestaltung des Biosensors ist die elektrische Erfassungsschaltung in einem Halbleiter-Chip integriert. Dies hat den Vorteil, dass dadurch der gesamte Messaufbau vereinfacht werden kann und eine höhere Messempfindlichkeit erreicht wird.

Eine weitere Vereinfachung ergibt sich bei dem Biosensor durch eine Ausgestaltung, bei der die Einheiten zum Immobilisieren auf dem Halbleiter-Chip angeordnet sind.

Bei dem Biosensor kann als Halbleiter-Chip prinzipiell jedes geeignete Halbleiter-Bauelement verwendet werden.

Vorzugsweise wird ein Transistorchip verwendet, der ein CMOS-Chip sein kann.

In einer Weiterbildung des Biosensors weist die elektrische Erfassungsschaltung einen Vorverstärker für die Verstärkung des erfassten Signals für jede Einheit zum Immobilisieren auf.

In einer anderen Ausgestaltung weist die elektrische Erfassungsschaltung eine Auswahlelektronik zum individuellen Auswählen mindestens einer Einheit zum Immobilisieren auf. Dies hat zum einen den Vorteil, dass dadurch Positionierungsprobleme bei der Beleuchtung des Sensors mit Licht, was für eine photoinduzierbare Messung erforderlich ist, vermieden werden, da die Positionsauswahl, d.h. welches Sensorfeld/welche Einheit zum Immobilisieren aktiviert wird, elektronisch geschieht. Insbesondere dies ermöglicht die Ausgestaltung des Biosensors als sogenanntes "handheld-Device" bzw. ermöglicht den (mobilen) Einsatz z.B. in Arztpraxen, Krankenhäusern, Notfall- oder Intensivmedizin oder im "Home-Care-Bereich". Zum anderen hat die Auswahlelektronik den Vorteil, dass das Übersprechen, d.h. der störende Einfluss von nicht ausgewählten Sensorpositionen, vollständig unterdrückt werden kann. Die maximale Anzahl von ansteuerbaren Sensorpositionen wird durch das Übersprechen nicht mehr begrenzt.

In einer weiteren Ausführungsform des Biosensors weist die elektrische Erfassungsschaltung einen Analog/Digital-Wandler für die Umwandlung des erfassten Signals für jede Einheit zum Immobilisieren auf. Dadurch kann eine Schnittstelle zu einer externen Elektronik digital ausgeführt werden und ist somit sehr unanfällig gegen elektromagnetische Störungen.

Als weitere Ausführungsform weist die elektrische Erfassungsschaltung des Biosensors eine Auswerteeinheit zur Auswertung des erfassten Signals für jede Einheit zum Immobilisieren auf. Unter einer Auswerteeinheit wird dabei eine Einheit verstanden, die ein eingehendes Messsignal weiterverarbeitet, indem sie das Signal z.B. zu einem anderen von der Einheit schon erfassten Signal addiert oder davon substrahiert, das erfasste Signal speichert, mit anderen Signalen vergleicht, und dadurch eine Information darüber erzeugt und ggf. anzeigt, ob ein Hybridisierungsereignis stattgefunden hat. Durch diese Auswerteeinheit wird somit eine "On-Chip-Signalverarbeitung" ermöglicht.

In einer anderen Ausgestaltung weist die elektrische Erfassungsschaltung des Biosensors für jede Einheit zum Immobilisieren eine Einheit zur Aufsummierung der an die jeweilige Einheit zum Immobilisieren abgegebene Ladungsmenge, d.h. einen Integrator, auf.

Ausführungsbeispiele der Erfindung sind in den Figuren dargestellt und werden im weiteren näher erläutert.

Es zeigen
- Figuren 1a und 1b: einen Biosensor der Erfindung zu unterschiedlichen Verfahrenszuständen;
- Figuren 2a bis 2d: das aus [1] bis [5] bekannte Verfahren für die Erfassung von Nukleinsäuren bzw. einen aus [1] bis [5] bekannten Biosensor;
- Figur 3: eine Vorrichtung mit einem hier beschriebenen Biosensor;

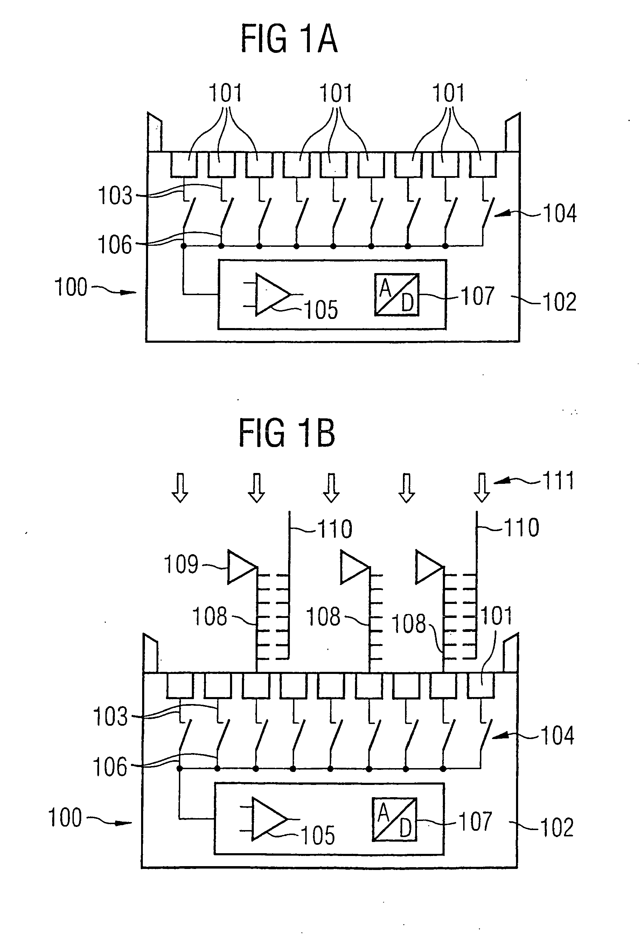

**Fig.1** zeigt eine Schnittansicht eines Biosensors 100 gemäß einem Ausführungsbeispiel des hier beschriebenen Biosensors.

**Fig.1a** zeigt den Biosensor 100 mit Einheiten zum Immobilisieren 101, die in einer Isolatorschicht 102 aus Isolatormaterial angeordnet sind.

Die Einheiten zum Immobilisieren 101 sind über elektrische Anschlüsse 103 mit einer elektrischen Erfassungsschaltung 104 verbunden. Die Einheiten zum Immobilisieren 101 sind aus Gold hergestellt.

Die elektrische Erfassungsschaltung 104 des Biosensors 100 weist einen Vorverstärker 105 für die Verstärkung des erfassten Signals für jede Einheit zum Immobilisieren, eine Auswahlelektronik 106 für das individuelle Auswählen von mindestens einer Einheit zum Immobilisieren sowie einen Analog/Digital-Wandler 107 für die Umwandlung des erfassten Signals für jede Einheit zum Immobilisieren auf.

Der Biosensor 100 kann in einem zweistufigen Verfahren erhalten werden, in dem er zunächst mittels eines Standardverfahren zur Herstellung von CMOS gefertigt werden und anschließend eine Goldschicht zur Ausbildung der Einheiten zum Immobilisieren auf den Chip aufgebracht wird.

Auf den Einheiten zum Immobilisieren 101 sind einzelsträngige DNA-Sondenmoleküle 108 aufgebracht, die eine redoxaktive Markierung 109 aufweisen (Fig.1b). Diese Markierung 109 kann z.B. ein photosynthetisches bakterielles Reaktionszentrum sein und wie in [3] auf Seiten 50, 4. Absatz bis Seite 52, 1. Absatz beschrieben, mit den Sondenmolekülen verknüpft werden (vgl. auch **Fig.2c).**

Zur Erfassung von Nukleinsäuren wird der Biosensor 100 in Kontakt mit einer zu untersuchenden Probe, etwa einem Elektrolyten (nicht gezeigt), gebracht.

**Fig.1b** zeigt den Biosensor 100 für den Fall, dass in dem Elektrolyten DNA-Stränge 110 enthalten sind, die eine vorgegebene Nukleotidsequenz aufweisen, die komplementär ist zu der Sequenz der DNA-Sondenmoleküle 108.

In diesem Fall hybridisieren die zu den DNA-Sondenmolekülen 108 komplementären DNA-Stränge 110 mit den DNA-Sondenmolekülen 108, die auf den Einheiten zum Immobilisieren 101 aufgebracht sind.

Wie aus **Fig.1b** ersichtlich ist, ist das Resultat nach erfolgter Hybridisierung, dass sich auf den Einheiten 101 hybridisierte Moleküle befinden, d.h. dort doppelsträngige DNA-Moleküle immobilisiert sind.

In einem weiteren Schritt kann gegebenenfalls mittels eines biochemischen Verfahrens, beispielsweise durch Zugabe von DNA-Nukleasen zu dem Elektrolyten, eine Hydrolyse von einzelsträngigen DNA-Sondenmoleküle 108 auf den Einheiten 101 bewirkt werden (vgl. **Fig.1b).**

Hierbei ist die Selektivität des abbauenden Enzyms für einzelsträngige DNA zu berücksichtigen. Besitzt das für den Abbau der nicht hybridisierten DNA-Einzelstränge ausgewählte Enzym diese Selektivität nicht, so wird möglicherweise die als doppelsträngige DNA vorliegende zu erfassende Nukleinsäure ebenfalls (unerwünschterweise) abgebaut, was zu einer Verfälschung des Messergebnisses führen würde.

Nach Entfernen von einzelsträngigen DNA-Sondenmolekülen, sind lediglich die Hybride aus den zu erfassenden DNA-Molekülen 110 und den dazu komplementären ersten DNA-Sondenmolekülen 108 vorhanden.

Beispielsweise kann zum Entfernen der nicht gebundenen einzelsträngigen DNA-Sondenmoleküle 108 auf Einheiten 101 zum Immobilisieren, einer der folgenden Stoffe zugegeben werden:
- Nuklease aus Mung-Bohnen,
- Nuklease P1, oder
- Nuklease S1.

Zu diesem Zweck können auch DNA-Polymerasen, die aufgrund ihrer 5'→ 3' Exonukleaseaktivität oder ihrer 3'→ 5' Exonukleaseaktivität imstande sind, einzelsträngige DNA abzubauen, verwendet werden.

Mittels einer nicht gezeigten Lichtquelle (z.B. eines Lasers) wird daraufhin durch Pfeile 111 symbolisiertes Licht mit einer Wellenlänge eingestrahlt, die geeignet ist, die Markierungen 109 wie z.B. ein bakterielles Reaktionszentrum anzuregen. Es kommt dadurch zu einer photoinduzierten Ladungstrennung innerhalb der Cofaktoren des Reaktionszentrums und zu einer intermolekularen Elektronenübertragung.

Liegt an den (durch die Auswahlelektronik bestimmten) Einheiten zum Immobilisieren 101 ein geeignetes Potential an, kommt es zu einer Übertragung eines Elektrons von den doppelsträngigen Hybridmolekülen auf die Einheiten 101, d.h. zu einem Stromfluss, der durch die elektrische Erfassungsschaltung 104 erfasst wird.

Auf diese Weise wird die Anwesenheit der DNA-Moleküle 110 ermittelt. Die Verwendung des hier beschriebenen Biosensors 100 erlaubt eine individuelle (und örtlich aufgelöste) Erfassung von einer oder mehreren Einheiten zum Immobilisieren und bietet neben einer gesteigerten Messempfindlichkeit eine deutliche Vereinfachung der gesamten Messanordnung.

**Fig.3** zeigt eine Vorrichtung 300 zur Erfassung von Nukleinsäuren, die einen Biosensor 301 aufweist, der entsprechend dem Biosensor gemäß Ausführungsbeispiel 1 aufgebaut ist. Dies heißt, der Biosensor 301 weist Einheiten zum Immobilisieren 302, die in einer Isolatorschicht 303 aus Isolatormaterial angeordnet sind.

Die Einheiten zum Immobilisieren 302 sind aus Gold hergestellt und über elektrische Anschlüsse 304 mit einer elektrischen Erfassungsschaltung 305 verbunden. Die elektrische Erfassungsschaltung 305 des Biosensors 301 weist einen Vorverstärker 306 für die Verstärkung des detektierte Signals für jede Einheit zum Immobilisieren, eine Auswahlelektronik 307 zum individuellen Auswählen mindestens einer Einheit zum Immobilisieren sowie einen Analog/Digital-Wandler 308 für die Umwandlung des detektierte Signal für jede Einheit zum Immobilisieren auf.

Die Vorrichtung 300 besitzt einen Träger 309, der den Sensor 301 hält und auf dem Sensor z.B. für die Probenvorbereitung oder das Auftragen von Fängermolekülen bewegt werden kann.

Ferner ist eine zu untersuchende Probe, ein flüssiger Analyt 310 auf dem Sensor aufgebracht.

Die Vorrichtung 300 weist darüber hinaus eine Lichtquelle 311 auf, durch die durch Pfeile 312 symbolisiertes Licht auf den Sensor gestrahlt werden kann. Die Lichtquelle 312 kann ebenfalls beweglich sein. Die Vorrichtung 300 weist schließlich noch eine nicht dargestellte Kontrolleinheit und z.B. Flüssigkeitsabgabemittel auf, mit denen ein Versuchsdurchgang mit z.B. Aufbringen der Fängermoleküle, Aufbringen von zu untersuchenden Lösungen, Entfernen der Lösungen und dgl. automatisiert werden kann.

In diesem Dokument sind folgende Veröffentlichungen zitiert:
[1] DE 199 01 761 A1
[2] DE 199 26 457 A1
[3] WO 00/42217 A1
[4] DE 199 21 940 A1
[5] WO 00/31101 A1
[6] US 5,653,939
[7] US 6,017,696

Bezugszeichenliste
- 100: Biosensor
- 101: Einheiten zum Immobilisieren
- 102: Isolatorschicht
- 103: elektrische Anschlüsse
- 104: elektrische Erfasssungsschaltung
- 105: Vorverstärker
- 106: Auswahlelektronik
- 107: Analog/Digitalwandler
- 108: DNA-Sondenmolekül
- 109: redoxaktive Markierung
- 110: DNA-Stränge

- 200: Sensor
- 201: leitfähige Oberflächenschicht
- 202: einzelsträngige Nukleinsäuremoleküle
- 203: redoxaktive Markierung
- 204: elektrischer Anschluss
- 205: DNA-Stränge
- 206: durch Pfeil symbolisiertes Licht
- 207: Pfeil
- 208: Test-Sites
- 209: Trägermaterial
- 210: Analyt

- 300: Vorrichtung zum Erfassen von Nukleinsäuren
- 301: Sensor
- 302: Einheiten zum Immobilisieren
- 303: Isolatorschicht
- 304: elektrische Anschlüsse
- 305: elektrische Erfassungsschaltung
- 306: Vorverstärker
- 307: Auswahlelektronik
- 308: Analog/Digital-Wandler
- 309: Träger
- 310: Analyt
- 311: Lichtquelle
- 312: durch Pfeil symbolisiertes Licht

## Patentansprüche

1. Verfahren zum Erfassen von Nukleinsäuren mittels mindestens zwei Einheiten zum Immobilisieren von Nukleinsäuren,
• bei dem die mindestens zwei Einheiten zum Immobilisieren von Nukleinsäuren jeweils elektrisch leitend ausgestaltet und voneinander elektrisch isoliert sind,
• bei dem die mindestens zwei Einheiten zum Immobilisieren von Nukleinsäuren mit als Fängermoleküle dienenden ersten Nukleinsäurenmoleküle versehen werden, wobei die ersten Nukleinsäuremoleküle als einzelsträngige Moleküle vorliegen und zweite zu erfassende Nukleinsäuremoleküle binden können, und wobei die ersten als Fängermoleküle dienenden einzelsträngigen Nukleinsäuremoleküle mit einer redoxaktiven Markierung versehen werden, die ein detektierbares Signal erzeugen kann,
• bei dem eine zu untersuchende Probe mit den mindestens zwei Einheiten zum Immobilisieren von Nukleinsäuren in Kontakt gebracht wird, wobei die zu untersuchende Probe die zweiten zu erfassenden Nukleinsäuremoleküle enthalten kann,
• bei dem in der zu untersuchenden Probe enthaltene zweite Nukleinsäuremoleküle an den Fängermolekülen gebunden werden, wodurch doppelsträngige Hybridmoleküle gebildet werden,
• bei dem die zweiten Nukleinsäuremoleküle mittels eines durch die redoxaktive Markierung verursachten Signals unter Verwendung einer in einem Halbleiter-Chip integrierten elektrischen Erfassungsschaltung erfasst werden, die derart ausgestaltet ist, dass sie die Elektronen, die im Fall eines Sensorereignisses von der Markierung freigesetzt werden, und welche von dem doppelsträngigen Hybrid aus Fängermolekülen und zu erfassenden Nukleinsäuremolekülen von der Markierung zur Oberfläche einer jeweiligen Einheit zum Immobilisieren geleitet werden, erfasst, und
• bei dem eine Einheit zum Immobilisieren zum individuellen Erfassen des Signals ausgewählt wird.

2. Verfahren nach Anspruch 1,
bei dem das durch die redoxaktive Markierung erzeugte Signal durch Messung eines Stromflusses, eines Widerstands oder einer Leitfähigkeit erfasst wird.

3. Verfahren nach Anspruch 2,
bei dem die redoxaktive Markierung eine photoinduzierbare redoxaktive Markierung oder eine chemisch induzierbare redoxaktive Markierung ist.

4. Verfahren nach Anspruch 3,
bei dem die photoinduzierbare redoxaktive Markierung ein photosynthetisches bakterielles Reaktionszentrum, ein Cyclophan oder ein wenigstens bimolekularer Elektronen-Donor/Elektronen-Akzeptor-Komplex ist.

5. Verfahren nach Anspruch 4,
bei dem der Elektronen-Donor und der Elektronen-Akzeptor des biomolekularen Elektronen-Donor/Elektronen-Akzeptor-Komplexes ein Charge-Transfer-Komplex oder ein Übergangsmetall-Komplex ist.

6. Verfahren nach einem der Ansprüche 1 bis 5,
bei dem als Nukleinsäuren DNA-Moleküle, RNA-Moleküle oder PNA-Moleküle erfasst werden.

7. Verfahren nach Anspruch 6,
• bei dem als Nukleinsäuremoleküle DNA- oder RNA-Einzelstränge mit einer vorgegebenen Nukleotidsequenz erfasst werden, und
• bei dem als Fängermoleküle DNA-Sondenmoleküle mit einer zu der vorgegebenen Nukleotidsequenz komplementären Nukleotidsequenz verwendet werden.

8. Verfahren nach Anspruch 7,
bei dem nicht gebundene DNA-Sondenmoleküle von den mindestens zwei Einheiten zum Immobilisieren entfernt werden.

9. Verfahren nach Anspruch 8,
bei dem zur Entfernung nicht gebundener DNA-Sondenmoleküle ein Enzym mit Nukleaseaktivität mit der Einheit zum Immobilisieren in Kontakt gebracht wird.

10. Verfahren nach Anspruch 9,
bei dem als Enzym mit Nukleaseaktivität mindestens einer der folgenden Stoffe verwendet wird:
• Nuklease aus Mung-Bohnen,
• Nuklease P1,
• Nuklease S1 oder
• DNA-Polymerasen, die aufgrund ihrer
5'-> 3' Exonukleaseaktivität oder ihrer
3'-> 5' Exonukleaseaktivität imstande sind, einzelsträngige DNA abzubauen.

11. Verfahren nach einem der Ansprüche 1 bis 10,
bei dem die Einheiten zum Immobilisieren Gold aufweisen.

12. Verfahren nach einem der vorhergehenden Ansprüche,
bei dem die mindestens zwei Einheiten zum Immobilisieren auf einem Halbleiter-Chip angeordnet sind.

13. Verfahren nach Anspruch 12,
bei dem der Halbleiter-Chip ein CMOS-Chip ist.

14. Biosensor zum Erfassen von Nukleinsäuren mit mindestens zwei Einheiten zum Immobilisieren von Nukleinsäuren und mit einer elektrischen Erfassungsschaltung,
• bei dem die mindestens zwei Einheiten zum Immobilisieren von Nukleinsäuren jeweils elektrisch leitend ausgestaltet und voneinander elektrisch isoliert sind,
• bei dem die mindestens zwei Einheiten zum Immobilisieren von Nukleinsäuren mit als Fängermoleküle dienenden ersten Nukleinsäuremoleküle versehen sind, wobei die ersten Nukleinsäuremoleküle als einzelsträngige Moleküle vorliegen und zweite zu erfassende Nukleinsäuremoleküle binden können, wobei die ersten als Fängermoleküle dienenden einzelsträngigen Nukleinsäuremoleküle mit einer redoxaktiven Markierung versehen sind, die ein detektierbares Signal erzeugen kann, und
• bei dem die elektrische Erfassungsschaltung derart ausgestaltet ist, dass die Erfassungsschaltung Nukleinsäuremoleküle, die an die Fängermoleküle gebunden haben, mittels der Markierung erfasst, wobei die Erfassungsschaltung derart ausgestaltet ist, dass sie die Elektronen, die im Fall eines Sensorereignisses von der Markierung freigesetzt werden, und welche von dem doppelsträngigen Hybrid aus Fängermolekülen und zu erfassenden Nukleinsäuremolekülen von der Markierung zur leitfähigen Oberfläche einer jeweiligen Einheit zum Immobilisieren geleitet werden, erfasst, und
• bei dem die elektrische Erfassungsschaltung eine Auswahlelektronik zum individuellen Auswählen mindestens einer Einheit zum Immobilisieren aufweist und bei dem die elektrische Erfassungsschaltung in einem Halbleiter-Chip integriert ist.

15. Biosensor nach Anspruch 14,
der mehrere Einheiten zum Immobilisieren von Nukleinsäuren in einer regelmäßigen Anordnung aufweist.

16. Biosensor nach Anspruch 15,
bei dem die Einheiten zum Immobilisieren auf dem Halbleiter-Chip angeordnet sind.

17. Biosensor nach Anspruch 16,
bei dem der Halbleiter-Chip ein CMOS-Chip ist.

18. Biosensor nach einem der Ansprüche 14 bis 17,
bei dem die elektrische Erfassungsschaltung einen Vorverstärker zum Verstärken des detektierten Signals für jede Einheit zum Immobilisieren aufweist.

19. Biosensor nach einem der Ansprüche 14 bis 18,
bei dem die elektrische Erfassungsschaltung einen Analog/Digital-Wandler zur Umwandlung des detektierten Signals für jede Einheit zum Immobilisieren aufweist.

20. Biosensor nach einem der Ansprüche 14 bis 19,
bei dem die elektrische Erfassungsschaltung eine Auswerteeinheit für das detektierte Signal für jede Einheit zum Immobilisieren aufweist.

21. Biosensor nach einem der Ansprüche 14 bis 20,
bei dem die elektrische Erfassungsschaltung für jede Einheit zum Immobilisieren eine Einheit zur Aufsummierung der an die jeweilige Einheit zum Immobilisieren abgegebenen Ladungsmenge aufweist.

## Claims

1. A method for detecting nucleic acids by means of at least two nucleic acid immobilization units,
- in which the at least two nucleic acid immobilization units are electrically conductive and electrically insulated from one another,
- in which the at least two nucleic acid immobilization units are provided with first nucleic acid molecules acting as scavenger molecules, the first nucleic acid molecules being present as single-stranded molecules and being capable of binding second nucleic acid molecules to be detected, and the first single-stranded nucleic acid molecules acting as scavenger molecules being provided with a redox-active label which can generate a detectable signal,
- in which a sample to be studied is brought in contact with the at least two nucleic acid immobilization units, wherein the sample to be studied may contain the second nucleic acid molecules to be detected,
- in which second nucleic acid molecules contained in the sample to be studied become bound to the scavenger molecules, so that double-stranded hybrid molecules are formed,
- in which the second nucleic acid molecules are detected by means of a signal caused by the redox-active label using an electrical detection circuit integrated in a semiconductor chip being configured to detect the electrons released from the label in the case of a sensor event and conducted by the double-stranded hybrid of scavenger molecules and nucleic acid molecules to be detected from the label to the surface of a respective immobilization unit, and
- in which an immobilization unit is selected for individual detection of the signal.

2. The method as claimed in claim 1, in which the signal generated by the redox-active label is detected by measuring a current flow, a resistance or a conductivity.

3. The method as claimed in claim 2, in which the redox-active label is a photo-inducible redox-active label or a chemically inducible redox-active label.

4. The method as claimed in claim 3, in which the photo-inducible redox-active label is a photosynthetic bacterial reaction center, a cyclophane or an at least bimolecular electron-donor/electron-acceptor complex.

5. The method as claimed in claim 4, in which the electron donor and the electron acceptor of the bimolecular electron-donor/electron-acceptor complex is a charge transfer complex or a transition metal complex.

6. The method as claimed in one of claims 1 to 5, in which DNA molecules, RNA molecules or PNA molecules are detected as the nucleic acids.

7. The method as claimed in claim 6,
- in which DNA or RNA single strands with a predetermined nucleotide sequence are detected as the nucleic acid molecules, and
- in which DNA probe molecules with a nucleotide sequence complementary to the predetermined nucleotide sequence are used as scavenger molecules.

8. The method as claimed in claim 7, in which unbound DNA probe molecules are removed from the at least two immobilization units.

9. The method as claimed in claim 8, in which an enzyme with nuclease activity is brought in contact with the immobilization unit in order to remove unbound DNA probe molecules.

10. The method as claimed in claim 9, in which at least one of the following substances is used as the enzyme with nuclease activity:
- mung bean nuclease,
- nuclease P1,
- nuclease S1, or
- DNA polymerases which are capable of degrading single-stranded DNA owing to their
5' -> 3' exonuclease activity or their
3' -> 5' exonuclease activity.

11. The method as claimed in one of claims 1 to 10, in which the immobilization units comprise gold.

12. The method as claimed in one of the preceding claims, in which the at least two immobilization units are arranged on a semiconductor chip.

13. The method as claimed in claim 12, in which the semiconductor chip is a CMOS chip.

14. A biosensor for detecting nucleic acids with at least two nucleic acid immobilization units and with an electrical detection circuit,
- in which the at least two nucleic acid immobilization units are electrically conductive and electrically insulated from one another,
- in which the at least two nucleic acid immobilization units are provided with first nucleic acid molecules acting as scavenger molecules, the first nucleic acid molecules being present as single-stranded molecules and being capable of binding second nucleic acid molecules to be detected, and the first single-stranded nucleic acid molecules acting as scavenger molecules being provided with a redox-active label which can generate a detectable signal, wherein the electrical detection circuit is configured to detect the electrons released from the label in the case of a sensor event and conducted by the double-stranded hybrid of scavenger molecules and nucleic acid molecules to be detected from the label to the conductive surface of a respective immobilization unit, and
- in which the electrical detection circuit has selection electronics for individual selection of at least one immobilization unit and in which the electrical detection circuit is integrated in a semiconductor chip.

15. The biosensor as claimed in claim 14, which has a plurality of nucleic acid immobilization units in a regular arrangement.

16. The biosensor as claimed in claim 15, in which the immobilization units are arranged on the semiconductor chip.

17. The biosensor as claimed in claim 16, in which the semiconductor chip is a CMOS chip.

18. The biosensor as claimed in one of claims 14 to 17, in which the electrical detection circuit has a preamplifier for preamplifying the detected signal for each immobilization unit.

19. The biosensor as claimed in one of claims 14 to 18, in which the electrical detection circuit has an analog/digital converter for converting the detected signal for each immobilization unit.

20. The biosensor as claimed in one of claims 14 to 19, in which the electrical detection circuit has an evaluation unit for the detected signal for each immobilization unit.

21. The biosensor as claimed in one of claims 14 to 20, in which the electrical evaluation unit for each immobilization unit has a unit for adding up the charge quantity imparted to the respective immobilization unit.

## Revendications

1. Procédé de détection d'acides nucléiques au moyen d'au moins deux unités d'immobilisation d'acides nucléiques,
• dans lequel les au moins deux unités d'immobilisation d'acides nucléiques sont, respectivement, conductrices de l'électricité et sont isolées électriquement les unes des autres ;
• dans lequel les au moins deux unités d'immobilisation d'acides nucléiques sont munies de premières molécules d'acides nucléiques servant de molécules de fixation, les premières molécules d'acides nucléiques se présentant sous la forme de molécules à brin simple ayant un marquage actif du point de vue redox, qui peut produire un signal pouvant être détecté ;
• dans lequel un échantillon à étudier est mis en contact avec les au moins deux unités d'immobilisation d'acides nucléiques, l'échantillon à étudier pouvant contenir les deuxièmes molécules d'acides nucléiques à détecter ;
• dans lequel des deuxièmes molécules d'acides nucléiques contenues dans l'échantillon à étudier sont fixées sur les molécules de fixation en formant des molécules hybrides à brin double ;
• dans lequel les deuxièmes molécules d'acides nucléiques sont détectées au moyen d'un signal provoqué par le marquage actif du point de vue redox en utilisant un circuit électrique intégré dans une puce à semi-conducteurs qui est tel qu'il détecte les électrons qui, dans le cas d'un événement de capteur, sont libérés du marquage et ceux de l'hybride à brin double constitués de molécules de fixation et de molécules d'acides nucléiques à détecter qui passent du marquage à la surface d'une unité respective d'immobilisation ; et
• dans lequel on choisit une unité d'immobilisation pour la détection individuelle du signal.

2. Procédé suivant la revendication 1,
dans lequel le signal produit par le marquage actif du point de vue redox est détecté par mesure d'un flux de courant, d'une résistance ou d'une conductivité.

3. Procédé suivant la revendication 2,
dans lequel le marquage actif du point de vue redox est un marquage actif du point de vue redox photo-inductible ou un marquage actif du point de vue redox pouvant être induit chimiquement.

4. Procédé suivant la revendication 3,
dans lequel le marquage actif du point de vue redox photo-inductible est un centre de réaction bactérien de photosynthèse, un cyclophane ou au moins un complexe donneur d'électrons/accepteur d'électrons au moins bimoléculaire.

5. Procédé suivant la revendication 4,
dans lequel le donneur d'électrons et l'accepteur d'électrons du complexe donneur d'électrons/accepteur d'électrons au moins bimoléculaire est un complexe à transfert de charge ou un complexe à métal de transition.

6. Procédé suivant l'une des revendications 1 à 5,
dans lequel on détecte comme acides nucléiques des molécules d'ADN, des molécules d'ARN ou des molécules d'APN.

7. Procédé suivant la revendication 6,
• dans lequel on détecte comme molécules d'acides nucléiques des brins individuels d'ADN ou d'ARN ayant une séquence prescrite de nucléotides ; et
• dans lequel on utilise comme molécules de fixation des molécules sondes d'ADN ayant une séquence de nucléotides complémentaire de la séquence de nucléotides prescrite.

8. Procédé suivant la revendication 7,
dans lequel on élimine les molécules sondes d'ADN non fixées des au moins deux unités d'immobilisation.

9. Procédé suivant la revendication 8,
dans lequel, pour éliminer les molécules sondes d'ADN non fixées, on met un enzyme ayant une activité de nucléase en contact avec l'unité d'immobilisation.

10. Procédé suivant la revendication 9,
dans lequel on utilise comme enzyme ayant une activité de nucléase au moins l'une des substances suivantes :
• de la nucléase d'ambérique
• de la nucléase P1 ;
• de la nucléase S1 ; ou
• des polymérases d'ADN qui, en raison de leur activité d'exonucléase 5' → 3' ou de leur activité d'exonucléase 3' → 5', sont en mesure de décomposer de l'ADN à brins simples.

11. Procédé suivant l'une des revendications 1 à 10,
dans lequel les unités d'immobilisation ont de l'or.

12. Procédé suivant l'une des revendications précédentes,
dans lequel les au moins deux unités d'immobilisation sont disposées sur une puce à semi-conducteurs.

13. Procédé suivant la revendication 12,
dans lequel la puce à semi-conducteurs est une puce CMOS.

14. Biocapteur de détection d'acides nucléiques comprenant au moins deux unités d'immobilisation d'acides nucléiques et un circuit électrique de détection,
• dans lequel les au moins deux unités d'immobilisation d'acides nucléiques sont, respectivement, conductrices de l'électricité et sont isolées électriquement les unes des autres ;
• dans lequel les au moins deux unités d'immobilisation d'acides nucléiques sont munies de premières molécules d'acides nucléiques servant de molécules de fixation, les premières molécules d'acides nucléiques se présentant sous la forme de molécules à brin simple ayant un marquage actif du point de vue redox qui peut produire un signal pouvant être détecté ; et
• dans lequel le circuit électrique de détection est tel qu'il détecte au moyen du marquage des molécules d'acides nucléiques qui ont été fixées aux molécules de fixation, le circuit de détection étant tel qu'il détecte les électrons qui, dans le cas d'un événement de capteur, ont été libérés par le marquage et ceux de l'hybride à brins doubles constitués de molécules de fixation et de molécules d'acides nucléiques à détecter passent du marquage à la surface conductrice d'une unité respective d'immobilisation ; et
• dans lequel le circuit électrique de détection comprend une électronique de sélection pour la sélection individuelle d'au moins une unité d'immobilisation et dans lequel le circuit électrique de détection est intégré dans une puce à semi-conducteurs.

15. Biocapteur suivant la revendication 14,
qui a plusieurs unités d'immobilisation d'acides nucléiques suivant un ordre régulier.

16. Biocapteur suivant la revendication 15,
dans lequel les unités d'immobilisation sont disposées sur la puce à semi-conducteurs.

17. Biocapteur suivant la revendication 16,
dans lequel la puce à semi-conducteurs est une puce CMOS.

18. Biocapteur suivant l'une des revendications 14 à 17,
dans lequel le circuit électrique de détection a un préamplificateur pour amplifier le signal détecté pour chaque unité d'immobilisation.

19. Biocapteur suivant l'une des revendications 14 à 18,
dans lequel le circuit électrique de détection a un convertisseur analogique/numérique de conversion du signal détecté pour chaque unité d'immobilisation.

20. Biocapteur suivant l'une des revendications 14 à 19,
dans lequel le circuit électrique de détection a une unité d'exploitation du signal détecté pour chaque unité d'immobilisation.

21. Biocapteur suivant l'une des revendications 14 à 20,
dans lequel le circuit électrique de détection a pour chaque unité d'immobilisation une unité pour faire la somme de la quantité de charge cédée à l'unité respective d'immobilisation.
